# EUROPEAN PATENT APPLICATION

(11) **EP 2 420 224 A1**
(43) Date of publication of application: **22.02.2012**
(21) Application number: 10172446.6
(22) Date of filing: 11.08.2010
(51) Int. Cl.: A61K 9/16, A61K 9/48

(54) **Method for producing a stable 3-(2,2,2-trimethylhydrazinium)propionate dihydrate solid pharmaceutical composition**

(71) Applicant: Grindeks, a joint stock company, 1057 Riga (LV)
(72) Inventor: The designation of the inventor has not yet been filed

(57) **Abstract**

Method for producing a stable pharmaceutical form of 3-(2,2,2-trimethylhydrazinium)propionate dihydrate and pharmaceutical composition thereof.

## Description

### Technical Field

The present invention relates to a method for producing a stable pharmaceutical composition of 3-(2,2,2-trimethylhydrazinium)propionate dihydrate and pharmaceutical composition thereof.

### Background Art

Active ingredient 3-(2,2,2-trimethylhydrazinium) propionate dihydrate is disclosed in WO 8001068 (A1) (ORCH SINTEZA; EREMEEV; LATVIETIS; GILLER; TRAPENTSIERS; SEMENIKHI) 29.05.1980 and it is well known that its International Nonproprietary Name is Meldonium dihydrate (registered with the trade mark of "MILDRONATS^{®} ", "MILDRONATE^{®}").

Most drug substances do not inherently have the physical and chemical characteristics that allow for direct processing into a tablet or capsule. Excipients are those ingredients added to the drug substance when formulating a drug product. Excipients are commonly classed as diluents, binders, flow aids or glidants, disintegrants, colorants, and lubricants.

Starch, which may come from corn, wheat or potatoes, is occasionally used as a tablet diluent. Potato starch is a fine, white, odourless, tasteless powder comprised of very small spherical or ovoid granules, whose size and shape are characteristic for each botanical variety.

Starch is used as an excipient primarily in oral solid-dosage formulations where it is utilized as a binder, diluent and disintegrant. In granulated formulations, about half of the total starch content is included in the granulation and the balance as part of final blend with the dried granulation.

Starch is the most commonly used tablet disintegrant at the level of 3 - 15 %.

Silica, colloidal anhydrous is a submicroscopic fumed silica with a particle size of about 15 nm. It is light, loose, bluish-white-coloured, odourless, tasteless, no gritty amorphous powder.

It is practically insoluble in organic solvents, water and acids, except hydrofluoric acid, soluble in hot solutions of alkali hydroxide. Forms a colloidal dispersion with water.

Silica, colloidal anhydrous is widely used in pharmaceuticals, cosmetics and food products as glidant.

Its small particle size and large specific surface area give it desirable flow characteristics that are exploited to improve the flow properties of dry powders in a number of processes such as tableting.

Calcium stearate is primarily used in pharmaceutical formulations as a lubricant in tablet and capsule manufacture at concentrations up to 1.0 % w/w. Although it has good antiaherent and lubricant properties, calcium stearate has poor glidant properties.

It is a fine, white to yellowish-white, bulky powder having a slight, characteristic odour. It is unctuous and free from grittiness.

It is practically insoluble in water, ethanol and ether.

It is primarily used as a lubricant in capsule and tablet manufacture at concentration between 0.25 % and 5.0 % m/m. It reduces the friction between the powder mixture and metallic parts of equipment during the compression.

As it is known the encapsulation of medical agents remains a popular method for administration of drugs because of:
1. Capsules are capable of masking the bitter taste or smell of various substances.
2. Hard gelatin capsules guarantee fast and reliable release of the active substance.
3. Capsulation ensures degree good accuracy and uniformity of dosage.
4. Some patients find it easier to swallow capsules than tablets, therefore preferring to take this form when possible.

The hard gelatin capsule, also referred to as the dry-filled capsule (dfc), consists of two sections, one slipping over the other, thus completely surrounding and protecting the drug formulation.

### Summary of invention

### Technical Problem

As it's well-known Meldonium as dihydrate has essential drawbacks, the first of which consists in its rather high hygroscopicity. Already after 24 hours maintenance at 100% air humidity, mass of Meldonium dihydrate is increased by 10 % because of water absorption, the substance being transformed into syrup.

Besides, Meldonium dihydrate is not very stable: while heated, it fast loses the water of crystal hydrate. In turn, the anhydrous form of Meldonium dihydrate is unstable and extremely hygroscopic. In such form, this compound soon becomes coloured and gets a specific annoying odour. Thus, the hygroscopicity and thermal non-stability of Meldonium dihydrate are significant disadvantages. Furthermore, Meldonium dihydrate is actively dehydrated at temperatures as low as 40-45°C. This means that sure storage of Meldonium dihydrate dosage forms containing crystal hydrate is rather embarrassing in countries with hot climate.

Meldonium dihydrate is relatively stable in its crystalline form but it has been shown that if Meldonium dihydrate solid pharmaceutical composition, such as capsule, is prepared by routine procedures it becomes unstable and its content in solid pharmaceutical composition, such as capsule, is likely to experience a time depending decrease.

It is object to the present invention to develop a method for providing a Meldonium dihydrate solid pharmaceutical form, such as capsule, preparation which is stable.

### Solution to Problem

Surprisingly it was found method of producing a stable, solid pharmaceutical composition of Meldonium dihydrate.

It is essentially important the way how solid pharmaceutical composition of Meldonium dihydrate is prepared.

The development of Meldonium dihydrate capsules by presented invention is based on direct fill technology.

In the method of producing solid pharmaceutical composition of Meldonium dihydrate, Meldonium dihydrate is used as an active ingredient, starch, preferably potato starch was used as diluent, silixon dioxide is used as a glidant and calcium stearate is used as lubricant.

During the manufacturing process a pre-mixture of the drug substance and excipients is prepared to ensure the content uniformity. The process was optimised to obtain a fast release of the active substance.

Meldonium dihydrate may be formulated in granules, capsules or the like.

The manufacturing process consists of the following phases:
1. Identification and checking of raw materials for release
2. Weighing of active ingredient and excipients
3. Blending of powder mass (active substance & excipients)
4. Lubrication of powder mass
5. In-process control
6. Capsule-filling

Method of producing Meldonium dihydrate is summarized in below scheme 1. 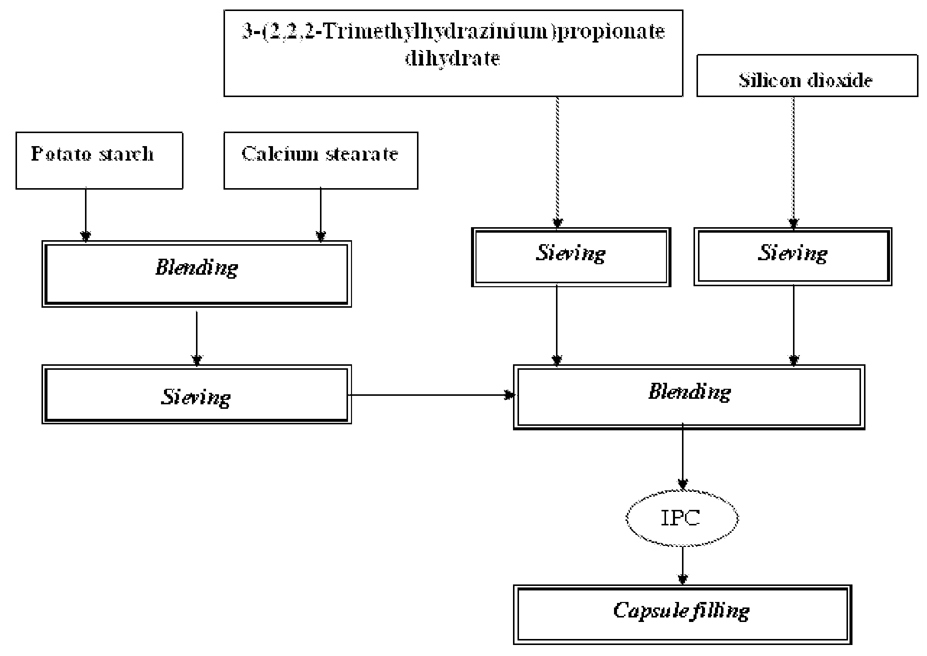 Scheme 1 method of producing Meldonium dihydrate solid pharmaceutical composition, such as capsule

### Best Mode for Carrying Out the Invention

The present invention will be described in detail through the following examples. The examples are presented for illustrating purposes only and should not be construed as limiting the invention.

**Table 1**

| Pharmaceutical composition of Meldonium dihydrate capsule | | | |
|---|---|---|---|
| Ingredients | mg | | Function |
| 3-(2,2,2-trimethylhydrazinium)propionate dihydrate | 250.0 | 500 | Active substance |
| Potato starch | 13.6 | 27.2 | disintegrant |
| Silicon dioxide | 5.4 | 10.8 | lubricant (glidant) |
| Calcium stearate | 2.7 | 5.4 | lubricant |

General method for producing Meldonium dihydrate capsule

After performing various trials the following procedure has been established:
Potato starch with calcium stearate are mixed;
active substance, previously prepared and screened mixture and
previously screened silicon dioxide mix.

During the manufacturing process a pre-mixture of the drug substance and excipients is prepared to ensure the content uniformity. The process was optimised to obtain a fast release of the active substance.

Method for producing Meldonium dihydrate compromise following steps:
(a) screening 3-(2,2,2-Trimethylhydrazinium)propionate dihydrate (250 mg) through stainless steel sieve;
(b) screening silixon dioxide (5.4 g) through nylon sieve;
(c) mixing separately potato starch (13.6 g) and calcium stearate (2.7 g);
(d) screening mixed potato starch and calcium stearate through nylon sieve;
(e) transferring screened Meldonium dihydrate, silixon dioxide and mixture of potato starch and calcium stearate into a mixer and mix;
(f) filling capsulating mass into capsules.

## Claims

1. A process for producing a stable, solid pharmaceutical form of Meldonium dihydrate which comprises:
(a) screening Meldonium dihydrate through stainless steel sieve;
(b) screening silixon dioxide through nylon sieve;
(c) mixing separately potato starch and calcium stearate;
(d) screening mixed potato starch and calcium stearate through nylon sieve;
(e) transferring screened Meldonium dihydrate, silixon dioxide and mixture of potato starch and calcium stearate into a mixer and mix;
(f) filling formed granulate into capsules.

2. A process according to claim 1, wherein solid pharmaceutical form is capsules, granules or the like.

3. A stable pharmaceutical composition, which comprises 250 mg Meldonium dihydrate as an active ingredient,
13.6 mg potato starch,
5.4 mg silicon dioxide,
2.7 mg calcium stearate.

4. A stable pharmaceutical composition, which comprises 500 mg Meldonium dihydrate as an active ingredient,
27.2 mg potato starch,
10.8 mg silicon dioxide,
5.4 mg calcium stearate.

5. A stable pharamceutical composition according to claim 3 or 4, wherein is oral dosage form.

6. A stable pharmaceutical composition according to claim 3 or 4, wherein oral doasge form is capsule.

7. A stable pharmaceutical composition according to claims 3-6 for use as a medicament.
